# NOUVEAU FASCICULE DE BREVET EUROPEEN

(11) **EP 2 651 978 B2**
(45) Date de publication et mention de la décision concernant l'opposition: **12.01.2022**
(45) Mention de la délivrance du brevet: 10.05.2017
(21) Numéro de dépôt: 11811089.9
(22) Date de dépôt: 12.12.2011
(51) Int. Cl.: C07K 16/28, A61K 39/395, A61P 35/00, A61P 37/00

(54) **UTILISATION D'UN ANTICORPS DIRIGE CONTRE UNE PROTEINE MEMBRANAIRE**
VERWENDUNG EINES ANTIKÖRPERS GEGEN EIN MEMBRANPROTEIN
USE OF AN ANTIBODY DIRECTED AGAINST A MEMBRANE PROTEIN

(30) Priorité: 13.12.2010 FR 1060428
(43) Date de publication de la demande: 23.10.2013
(60) Demande divisionnaire: 17159253.8
(73) Titulaire: Laboratoire Français du Fractionnement et des Biotechnologies, 91940 Les Ulis (FR)
(72) Inventeur: FOURNIER, Nathalie, F-59193 Erquinghem-Lys (FR); DE ROMEUF, Christophe, F-59130 Lambersart (FR)
(74) Mandataire: Plasseraud IP
(86) Numéro de dépôt international: PCT/FR2011/052949
(87) Numéro de publication internationale: WO 2012/080642

(56) Documents cités:
- EP-B1- 1 301 539
- WO-A1-01/38496
- WO-A1-2008/137382
- WO-A1-2014/093396
- WO-A2-01/36487
- WO-A2-2004/024768
- US-A1- 2010 189 641
- US-A1- 2018 066 060
- US-A1- 2018 086 834
- GARNACHE-OTTOU FRANCINE ET AL: "Identification of BDCA-2 and high levels of CD123 expression as useful markers for the diagnosis of plasmacytoid dendritic cell leukemia", ASH Annual Meeting Abstracts BLOOD, vol. 106, no. Abst. 3269 novembre 2005 (2005-11), XP002670970, Extrait de l'Internet: URL:http://abstracts.hematologylibrary.org /cgi/content/abstract/106/11/3269?maxtosho w=&hits=50&RESULTFORMAT=&searchid=1&FIRSTI NDEX=150&displaysectionid=Poster+Sessions& fdate=1/1/2005&tdate=12/31/2005&resourcety pe=HWCIT [extrait le 2012-03-07]
- JAYE DAVID L ET AL: "Expression of the plasmacytoid dendritic cell marker BDCA-2 supports a spectrum of maturation among CD4+CD56+hematodermic neoplasms", MODERN PATHOLOGY, vol. 19, no. 12, 1 septembre 2006 (2006-09-01), pages 1555-1562, XP002481366, NATURE PUBLISHING GROUP, US ISSN: 0893-3952, DOI: 10.1038/MODPATHOL.3800679
- LONARDI S ET AL: "A monoclonal antibody anti-BDCA2 (CD303) highly specific for plasmacytoid dendritic cell neoplasms on paraffin sections", VIRCHOWS ARCHIV, vol. 455, no. Suppl. 1, août 2009 (2009-08), pages 254-255, XP002644181, & 22ND EUROPEAN CONGRESS OF PATHOLOGY; FLORENCE, ITALY; SEPTEMBER 04 09, 2009 ISSN: 0945-6317
- CHAPEROT L ET AL: "Identification of a leukemic counterpart of the plasmacytoid dendritic cells", BLOOD, AMERICAN SOCIETY OF HEMATOLOGY, US, vol. 97, no. 10, 15 May 2001 (2001-05-15), pages 3210-3217, XP002243855, ISSN: 0006-4971, DOI: 10.1182/BLOOD.V97.10.3210
- ZÖLLER M ET AL: "IgG- and IgM-induced cellular cytotoxicity.", SCANDINAVIAN JOURNAL OF IMMUNOLOGY NOV 1982, vol. 16, no. 5, November 1982 (1982-11), pages 379-388, ISSN: 0300-9475
- N. KARAGIANNIS ET AL: "Activity of human monocytes in IgE antibody-dependent surveillance and killing of ovarian tumor cells", EUROPEAN JOURNAL OF IMMUNOLOGY, vol. 33, no. 4, 1 April 2003 (2003-04-01), pages 1030-1040, XP055038303, ISSN: 0014-2980, DOI: 10.1002/eji.200323185
- FRANKEL ARTHUR E ET AL: "Activity of SL-401, a targeted therapy directed to interleukin-3 receptor, in blastic plasmacytoid dendritic cell neoplasm patients.", BLOOD 17 JUL 2014, vol. 124, no. 3, 17 July 2014 (2014-07-17) , pages 385-392, ISSN: 1528-0020
- FEUILLARD JEAN ET AL: "Clinical and biologic features of CD4(+)CD56(+) malignancies.", BLOOD 1 MAR 2002, vol. 99, no. 5, 1 March 2002 (2002-03-01), pages 1556-1563, ISSN: 0006-4971
- GARNACHE-OTTOU: "Une nouvelle entité: les leucémies CD4+ CD56+ dérivées des cellules dendritiques plasmocytoïdes", SCTRA BIOLOGIE, vol. 147, 1 September 2005 (2005-09-01), pages 49-58,
- CHAPEROT et al.: "Leukemic plasmacytoid dendritic cells share phenotypic and functional features with their normal counterparts", Eur. J. Immunol., vol. 34, no. 2, 1 February 2004 (2004-02-01), pages 418-426,
- MITCHEL L et al.: "Depletion of B Cells In Vivo by a Chimeric Mouse Human Monoclonal Antibody to CD 20", Blood, vol. 83, no. 2, 15 January 1994 (1994-01-15), pages 435-445,
- WINKLER et al.: "Cytokine-Release Syndrome in Patients With B- Cell Chronic Lymphocytic Leukemia and High Lymphocyte Counts After Treatment With an Anti- CD 20 Monoclonal Antibody (Rituximab, IDEC-C2B8", Blood, vol. 94, no. 7, 1999, pages 2217-2224,
- WEINER et al.: "Rituximab: mechanism of action", Semin Hematol, vol. 47, no. 2, April 2010 (2010-04), pages 115-123,
- NESTLE et al.: "Plasmacytoid predendritic cells initiate psoriasis through interferon-alpha production", JEM, vol. 202, no. 1, 4 July 2005 (2005-07-04), pages 135-143,
- DZIONEK et al.: "BDCA-2, BDCA-3, and BDCA-4: Three Markers for Distinct Subsets of Dendritic Cells in Human Peripheral Blood", The Journal of Immunology, vol. 165, 2000, pages 6037-6046,
- DZIONEK et al.: "BDCA-2, a Novel Plasmacytoid Dendritic Cell -specific Type II C-type Lectin, Mediates Antigen Capture and Is a Potent Inhibitor of Interferon alpha/beta Induction", JEM, vol. 194, no. 12, 17 December 2001 (2001-12-17), pages 1823-1834,
- FACHETTI et al.: "Blastic plasmacytoid dendritic cell neoplasm", Hematology Meeting Reports, vol. 3, no. 3, 2009, pages 1-3,
- GARNACHE-OTTOU et al.: "Extended diagnostic criteria for plasmacytoide dendritic cell leukaemia", British Journal of Hematology, vol. 145 , pages 624-636,
- PETRELLA et al.: "Tumoral aspects of plasmacytoid dendritic cells: What do we know in 2009?", Autoimmunity, vol. 43, no. 3, May 2010 (2010-05), pages 210-214,
- CHARLES et al.: "Plasmacytoid dendritic cells and dermatological disorders: focus on their role in autoimmunity and cancer", Eur J Dermatol, vol. 20, no. 1, 2010, pages 1-8,
- DZIONEK et al.: "Plasmacytoid Dendritic Cells: From Specific Surface Markers to Specific Cellular Functions", Human Immunology, vol. 63, 2002, pages 1133-1148,
- Zola et al: "CD molecules 2005: human cell differentation molecules", Blood, vol. 106, no. 9, 1 November 2005 (2005-11-01),
- Bosley, A: "Anticorps monoclonal anti-CD20 dans les maladies hématologiques et les affections auto-immunes", Réanimation, vol. 15, 2007, pages 270-277,
- Cao et al: "Plamacytoid dendritic cell-specific receptor ILT7-FceRIy inhibits Toll-like receptor-induced interferon production", The Journal of Experimental Medicine, vol. 203, no. 6, 12 June 2006 (2006-06-12) , pages 1399-1405,
- Graziano et Guyre: "Antibody-dependant Cellmediated Cytotoxicity (ADCC)", Encyclopedia of Life Sciences, 2006,
- Ludwig et al: "Monoclonal antibody therapeutics and apoptosis", Oncogene, vol. 22, 2003, pages 9097-9106,
- Natsume et al: "Engineered Antibodies of IgGl/lgG3 Mixed Isotope with Enhanced Cytotoxic Activities", Cancer Res., vol. 68, no. 10, 15 May 2008 (2008-05-15),
- Zöller et al: "IgG- and IgM-induced cytotoxicity", Scand. J. Immunol., vol. 16, 1982, pages 379-388,
- Karagiannis et al: "Activity of human monocytes in IgE antibodydependent surveillance and killing of ovarian tumor cells", Eur. J. Immunol., vol. 33, 2003, pages 1030-1040,
- Maeda et al: "A novel plasmacytoid dendritic cell line, CAL-1, established from a patient with blastic natural killer cell lymphoma", Int J Hematol., vol. 81, no. 2, February 2005 (2005-02), pages 148-154,
- Hansel et al: "The safety and side effects of monoclonal antibodies", Nature Reviews Drug Discobery, vol. 9, 2010, pages 325-338,
- Kovtun et al: "A CD123-targeting antibody-drug conjugate, IMGN632, designed to eradicate AML while sparing normal bone marrow cells", Blood Advances, vol. 2, 2018, pages 848-858,
- De Romeuf et al: "Chronic lymphocytic leukaemia cells are efficiently killed by an anti-CD20 monoclonal antibody selected for improved engagement of FcgammaRIII/CD16.", British Journal of Haematology, vol. 140, 2008, pages 635-643,
- Testa et al: "CD123 is a membrane biomarker and a therapeutic target in hematologic malignancies", Biomarker Research, vol. 2, no. 4, 2014,
- Legrand et al: "Functional CD47/signal regulatory protein alpha (SIRPalpha) interaction is required for optimal human T- and natural killer-(NK) cell homeostasis in vivo", PNAS, vol. 108, no. 32, 2011, pages 13224-13229,

## Description

La présente invention a pour objet l'utilisation d'un anticorps dirigé contre une protéine membranaire, et notamment pour le traitement de pathologies.

Les anticorps utilisés en clinique, dans le but de réguler une pathologie auto-immune ou inflammatoire, sont à ce jour principalement les anticorps anti-CD20 et en particulier l'anti-CD20 Rituxan. Le problème associé à ce type de traitement est du au fait que les anti-CD20 vont éliminer l'ensemble des cellules pré-B et lymphocytes B matures, mais également environ 20% des plasmocytes et une population de lymphocytes B mémoire (Gonzales-Stawinski Clin Immunol 2001, 98, 175 ; Looney, Ann rheum dis 2002, 61, 863) pouvant ainsi conduire à un état immuno-déficient associé à une hypogammaglobulémie. Il existe donc un besoin de trouver une alternative à cette déplétion B en diminuant le nombre de cellules dendritiques plasmacytoïdes, cellules clés à l'initiation de la réponse immune pathologique, et donc atténuer la réponse immune du patient et par conséquence de cellules B auto-réactives.

Les cellules dendritiques (en anglais dendritic cells ou DC) sont des cellules du système immunitaire présentatrices d'antigènes (CPA) faisant partie du système réticulohistiocytaire. Sous certaines conditions, les cellules DC présentent des prolongements cytoplasmiques similaires aux dendrites des neurones.

Les cellules dendritiques ont deux fonctions principales :
- le déclenchement de la réponse immunitaire adaptative, dont les acteurs principaux sont les lymphocytes T et les lymphocytes B, dirigée contre des antigènes du « non-soi ».
- le maintien de la tolérance centrale au « soi » dans le thymus, par le processus impliquant les lymphocytes T dit de sélection négative.

De nombreuses sous populations de cellules DC ont été caractérisées chez la souris, sans pour autant qu'il ait été identifié de correspondance chez l'homme. Les DC sont capables de se différencier en divers sous-types, selon les stimuli qu'elles reçoivent. Il existe deux grands types de cellules DC : les cellules DC conventionnelles, les cellules DC plasmacytoïdes et les cellules DC inflammatoires.

Les cellules DC conventionnelles comprennent :
- les cellules DC migratoires qui résident, à l'état basal, en périphérie. Suite à la phagocytose d'une particule antigénique et/ou à la réception de signaux, elles migrent vers les ganglions lymphoïdes secondaires par l'intermédiaire des vaisseaux lymphatiques ; elles arrivent à l'état mature dans les organes lymphoïdes où elles présentent l'antigène aux lymphocytes T naïfs ; on peut citer par exemple des cellules de Langerhans ou encore les cellules D des muqueuses ;
- les cellules DC immatures résidentes des organes lymphoïdes ; ces cellules collectent et présentent les antigènes du soi ou étrangers au sein même des organes lymphoïdes ; il s'agit de la plupart des cellules DC du thymus ou de la rate.

Les cellules DC inflammatoires sont recrutées dans les tissus suite à une inflammation où une infection mais ne sont jamais présentes en dehors de toute stimulation. On suppose actuellement que les cellules DC inflammatoires seraient principalement issues de la différenciation des monocytes sanguins.

Les cellules DC plasmacytoïdes diffèrent des cellules DC conventionnelles en ce qu'elles sont circulantes, rondes et sans dendrites à l'état basal, bien qu'elles finissent par se différencier en DC conventionnelles après activation. Elles sont donc capables de présenter l'antigène.
Après stimulation, en général par un antigène viral, elles produisent une grande quantité d'interférons (IFN) de classe I. Ces cellules sont essentiellement impliquées dans la réponse anti-virale et dans les désordres auto-immuns.

Les cellules DC plasmacytoïdes (aussi appelées cellules DC plasmacytoïdes) ont été caractérisées phénotypiquement : elles expriment les marqueurs CD4 et BDCA-2 et 4. Leur phénotype est donc CD4+, CD11c-, Lin-, BDCA-2+, BDCA-4+.

Les cellules DC plasmacytoïdes peuvent être à l'origine de tumeurs hématopoïétiques dans lesquelles elles acquièrent un marqueur supplémentaire qui est le CD56+. C'est pourquoi on parle de tumeurs hématopoïétiques CD4+/CD56+ (en anglais BPDCN : Blastic plasmacytoid dendritic cells neoplasm).

Les tumeurs hématopoïétiques CD4+/CD56+ sont des néoplasmes hématologiques rares (1% des leucémies aigues), qui se présentent sous la forme de nodules cutanés associés à une lympho-adénopathie ou un gonflement de la rate et à une cytopénie fréquente. Ces manifestations cutanées sont très rapidement suivies par une infiltration de la moelle osseuse. Du fait de l'expression du marqueur CD56+, ces pathologies ont d'abord été classées dans les lymphomes à cellules NK. Mais l'absence de marqueurs de lignées myéloïdes, lymphoïdes T ou B ou NK a suscité des études complémentaires qui ont permis d'affiner la classification pour enfin révéler que les cellules dendritiques dite de type II ou cellules DC plasmacytoïdes sont à l'origine des tumeurs hématopoïétiques CD4+/CD56+*[*Chaperot L et al., 2001, Blood. 2001 May 15;97(10):3210-7, Herling M, Jones D., 2007, Am J Clin Pathol. 2007 May; 127(5):687-700*]*

La prise en charge actuelle de ces pathologies est basée sur la chimiothérapie qui permet une rémission complète pour 2 patients sur trois environ mais où les rechutes sont fréquentes et précoces (environ 9 mois). La médiane de survie globale est de 13 mois environ. Une autre alternative de traitement est l'allogreffe de cellules hématopoïétiques qui ne permet néanmoins pas non plus de survie à long terme.

En conséquence, il existe à ce jour un réel besoin de trouver un traitement à ces pathologies CD4+/CD56+.

Des moyens de traitement de ces pathologies impliquant des cellules exprimant des marqueurs des cellules dendritiques sont proposés dans l'art antérieur. Notamment, des molécules ciblant les marqueurs de différentiation BDCA ont été proposées.

On peut citer par exemple le brevet européen EP 1 301 539 qui décrit un polypeptide de séquence particulière se liant spécifiquement à la protéine BDCA-2 et une composition pharmaceutique le comprenant.
La demande de brevet européen EP 1 783 141 divulgue une composition permettant d'induire une réponse immunitaire chez un patient, ladite composition comprenant des cellules dendritiques préalablement traitées avec un anticorps anti-BDCA-2 et exprimant un antigène (tumoral, viral, bactérien...). Ceci consiste en un traitement par thérapie cellulaire.
La demande WO 01/365487 décrit des anticorps monoclonaux dirigés contre la protéine BDCA-2, et en particulier les clones AC144, AD5-13A11 et AD5-4B8, ainsi que des fragments dérivés. Par ailleurs, cette demande décrit l'utilisation des anticorps monoclonaux pour le traitement de pathologies telles que les infections virales, les maladies auto-immunes et les tumeurs.
La demande WO 2006/037247 décrit l'utilisation d'anticorps monoclonaux dirigés contre la protéine BDCA-2, dans le cadre du traitement d'une maladie auto-immune particulière : le psoriasis.
Nestle et al., (Nestle et al. 2005, J. Exp. Med, vol 202, n°1, pp : 135-143) enseigne que des anticorps dirigés contre la protéine BDCA-2 peuvent être injectés par voie intraveineuse, dans un but thérapeutique dans le cadre du traitement du psoriasis.

Blomberg et al., (Blomberg et al., 2003, Arthritis and Rheumatism, vol 48, n°9, pp : 2524-2532) enseigne l'utilisation d'anticorps dirigés contre la protéine BDCA-2, lesdits vaccins étant utilisés pour d'une maladie auto-immune : le Lupus Erythémateux. Les auteurs montrent que lesdits anticorps sont capables d'inhiber la production d'interféron α (IFN-α). La demande US 2010/1896641 décrit des anticorps dirigés contre la protéine BDCA-2 dans le cadre du traitement des tumeurs.

Le document WO 01/38496 décrit le traitement de néoplasmes cutanés CD4+/CD56+ à l'aide d'anticorps dirigés contre des monocytes plasmacytoïdes CD56+.

On constate donc que l'utilisation d'anticorps dirigés contre la protéine BDCA-2 est largement décrite dans l'art antérieur.

Toutefois, l'art antérieur est muet quant au traitement de pathologies impliquant des cellules CD4+/CD56+.

BDCA-2 est également décrit comme marqueur cellulaire (voir par exemple Garnache-Ottou et al., BLOOD, vol. 106, n° 11, Part 1, 2005-11, page 941A; Leonardi et al, Virchows Archiv, vol 455, n°. Suppl. 1, 2009-08, pages 254-255 ; ou Jaye et al, Modem Pathology, vol. 19, n° 12, pages 1555-1562).

Cependant, à ce jour il n'existe aucun traitement spécifique et efficace permettant le traitement ou la prévention de pathologies impliquant des cellules dendritiques plasmacytoïdes, et notamment les tumeurs CD4+/CD56+.

L'invention a donc pour objet de palier le manque de l'art antérieur, et notamment de fournir des traitements alternatifs.
De plus, l'invention a pour but de fournir un nouveau traitement efficace et spécifique des tumeurs à cellules CD4+/CD56+.
L'invention a également pour objet des compositions médicamenteuses en vue de traiter et/ou prévenir lesdites pathologies.

L'invention concerne un anticorps monoclonal ou polyclonal dirigé contre la protéine BDCA-2 pour son utilisation dans le cadre de la prévention ou du traitement des pathologies impliquant une activation des cellules dendritiques plasmacytoïdes. L'invention repose sur la constatation inattendue faite par les Inventeurs de l'effet d'anticorps anti-BDCA-2 sur la progression de tumeurs, notamment des tumeurs hématopoïétiques.
L'invention se distingue de l'art antérieur en ce qu'elle implique une destruction des cellules DC plasmacytoïdes par apoptose ou par le mécanisme de l'ADCC.

L'utilisation d'anticorps anti-IFN-α dans le traitement des patients atteints de pathologies auto-immunes et inflammatoires induit une neutralisation de l'IFN-α d'une façon systémique, ce qui augmente potentiellement le risque d'infections opportunistes. En effet cette cytokine est produite en réponse à un agent infectieux en particulier viral.
Pour répondre à cette problématique, l'objet ici décrit et non revendiqué consiste à éliminer les cellules DC plasmacytoïdes, source principale d'IFN-α, recrutées au niveau des lésions inflammatoires et responsable de l'inflammation locale, ceci étant particulièrement bien décrit dans le cas du psoriasis par exemple. L'avantage réside donc en une élimination de l'IFN-α préférentiellement au lieu de l'inflammation et non de façon systémique, ceci diminuant par fait les risques liés à l'immunodéficience induits par les anti-IFN-α.
Un avantage certain et additionnel réside en l'absence d'effet sur les leucocytes (Papot, 2002, rev med interne, 23, supp 4), les macrophages (Levesque MC, 1999, Ar-thritis Rheum, 42, 569) et les fibroblastes (Houglum, JE, 1893, 2,20) et ainsi de préserver les sources d'IFN-α produites par ces types cellulaires.

Un autre avantage consiste *via* l'élimination des cellules DC plasmacytoïdes est la diminution d'autres cytokines pro-inflammatoires comme l'IL-6 également sécrétées par la cellules DC plasmacytoïdes dans un environnement inflammatoire et conduisant à une réponse Th1 par exemple.

De plus, contrairement aux concepts généralement admis, l'objet de la divulgation non revendiqué consiste à prendre avantage de l'hyper-activation du système immunitaire au site de l'inflammation, en particulier l'activation des cellules effectrices dont les cellules NK et les macrophages et cela du à la présence de nombreuses cytokines et chimiokines pro-inflammatoires pour obtenir *in fine* une diminution de l'inflammation. En effet, les auteurs ont montré que l'anticorps anti-BDCA-2 cité avait une meilleure activité cytotoxique contre les pDC en présence de molécules dont les taux sont augmentés dans les pathologies auto-immunes et inflammatoires (ie IFN-γ, TNF-α, ...).

La protéine BDCA-2 est exprimée de manière spécifique à la surface des cellules DC plasmacytoïdes, et est une protéine de type II appartenant aux lectines de type C.
Dans le cadre de la présente invention, le terme « anticorps » se réfère à une immunoglobuline, protéine constituée de 4 chaînes participant à la réponse immunitaire acquise. Les immunoglobulines sont bien connues de l'homme de métier et sont constituées d'un assemblage de deux dimères constitués chacun d'une chaîne lourde et d'une chaîne légère. Le complexe multimérique est assemblé par la liaison d'une chaîne légère et d'une chaîne lourde par un pont disulfure entre deux cystéines, les deux chaînes lourdes étant elle-même également reliées entre elles par deux ponts disulfures.

Chacune des chaînes lourdes et des chaînes légères est constituée d'une région constante et d'une région variable. L'assemblage des chaînes qui composent un anticorps permet de définir une structure tridimensionnelle caractéristique en Y, où
- la base du Y correspond à la région constante Fc qui est reconnue par le complément et les récepteurs Fc, et
- l'extrémité des bras du Y correspondent à l'assemblage respectif des régions variables de la chaîne légère et variable de la chaîne lourde qui sont reconnues par un antigène spécifique.

Plus précisément, chaque chaîne légère est constituée d'une région variable (V_{L}) et d'une région constante (C_{L}). Chaque chaîne lourde est constituée d'une région variable (V_{H}) et d'une région constante constituée de trois domaines constants C_{H1}, C_{H2} et C_{H3}. Les domaines C_{H2} et C_{H3} composent le domaine Fc.

Les anticorps décrits dans l'invention sont isolés et purifiés, et sont différents des anticorps naturels. Ces anticorps sont matures, c'est-à-dire qu'ils possèdent une structure tridimensionnelle ad hoc leur permettant de reconnaitre l'antigène, et possèdent toutes les modifications post-traductionnelles essentielles à leur reconnaissance antigénique.
Dans un aspect de l'invention, les anticorps sont polyclonaux.
Dans un autre aspect, il s'agit d'anticorps monoclonaux, c'est-à-dire qu'ils ne reconnaissent qu'un seul déterminant antigénique dans BDCA-2, contrairement aux anticorps polyclonaux qui correspondent à un mélange d'anticorps monoclonaux, et donc peuvent reconnaitre plusieurs déterminants antigéniques dans une même protéine.
Les anticorps monoclonaux de l'invention peuvent être obtenus par des techniques bien connues de l'homme de métier, et notamment par la technique de fusion cellulaire, ou encore la technique de clonage des séquences des chaines lourdes et légères, par technique de phage ou ribosome display, par immunisation de souris ayant le répertoire des immunoglobulines humaines et expression dans une cellule ad hoc ou un animal transgénique. Ces techniques sont bien connues de l'homme de métier.

Par cellules dendritiques plasmacytoïdes, on entend la sous population de cellules dendritiques aussi appelée DC2 caractérisée par les marqueurs HLA-DR+, CD11c-, CD123+ et CD45RA+, présentes dans les organes lymphoïdes et également en circulation dans le sang et caractérisées par leur capacité à sécréter de l'IFN de type I en présence d'une infection virale.

Dans l'invention, on entend par activation des cellules dendritiques plasmacytoïdes les différents mécanismes ayant pour effet d'activer la prolifération, la sécrétion cytokine déterminées, notamment les interférons de classe I, et la modification phénotypiques et morphologiques des cellules plasmacytoïdes.

L'invention est illustrée par un anticorps monoclonal ou polyclonal dirigé contre la protéine BDCA-2 pour son utilisation définie précédemment, où lesdites pathologies impliquant des cellules dendritiques plasmacytoïdes sont choisies parmi les tumeurs. Les maladies auto-immunes et les maladies inflammatoires ne font pas l'objet de l'invention ici revendiquée.

L'invention concerne un anticorps monoclonal ou polyclonal dirigé contre la protéine BDCA-2 pour son utilisation susmentionnée, où les tumeurs sont des tumeurs hématopoïétiques.
Dans l'invention, on entend par tumeurs hématopoïétiques des tumeurs ou maladies tumorales impliquant des cellules de la lignée sanguine, ou cellules hématopoïétiques. Ces tumeurs peuvent également être appelées hémopathies malignes.
Les hémopathies malignes embrassent
- les leucémies : tumeur où les cellules sanguines prolifèrent anormalement dans le sang,
- les lymphomes : tumeur cellules sanguines prolifèrent anormalement dans les organes lymphoïdes secondaires (ganglions, rate...)
- ou la Maladie de Kahler : tumeur où les cellules sanguines prolifèrent anormalement la moelle osseuse.

L'invention concerne un anticorps monoclonal ou polyclonal dirigé contre la protéine BDCA-2 pour son utilisation telle que définie précédemment, dans le cadre de la prévention ou du traitement des pathologies impliquant une activation des cellules dendritiques plasmacytoïdes, où lesdites pathologies sont des tumeurs hématopoïétiques de phénotype CD4+, CD56+.

On entend par tumeurs hématopoïétiques CD4+/CD56+, des hémopathies malignes dans lesquelles les cellules cancéreuses expriment conjointement les deux marqueurs CD4 et CD56. Ces maladies embrassent les lymphomes cutanés agranulaire CD4+/CD56+, les hématodermies agranulaires CD4+/CD56+, les leucémies/lymphomes NK blastiques, les lymphomes NK [Ng AP et al. 2006. Haematologica 91 (1): 143-4; Kim Y et al. 2005 J. Korean Med. Sci. 20 (2): 319-24; Chan JK, et al. 1997 Blood 89 (12): 4501-13]

La divulgation non revendiquée est également illustrée par un anticorps monoclonal ou polyclonal dirigé contre la protéine BDCA-2 pour son utilisation telle que définie ci-dessus, où les maladies auto-immunes et inflammatoires sont choisies parmi la maladie des tissus conjonctifs, les différents type de scléroses, l'inflammation autoimmune pulmonaire, le syndrome Guillain-Barre, la thyroidite autoimmune, le mellitis, le myasthenia gravis, la maladie du greffon contre l'hôte, la maladie autoimmune inflammatoire de l'oeil, le psoriasis, la maladie de Basedow (hyperthyroïdie), la thyroïdite chronique de Hashimoto (hypothyroïdie), le lupus érythémateux disséminé (LED), le syndrome de Goodpasture, le pemphigus, la Myasthénie, le diabète par insulinorésistance, l'anémie hémolytique auto-immune, le purpura thrombocytopénique auto-immun, la polyarthrite rhumatoïde, la sclérodermie, polymyosite et dermatomyosite, l'anémie de Biermer, la maladie de Gougerot-Sjogren, la glomérulonéphrite, la maladie de Wegener, la maladie de Horton, la périartérite noueuse et le syndrome de Churg et Strauss, la maladie de Still, la polychondrite atrophiante, la maladie de Be-hçet, la sclérose en plaques, la spondylarthrite, la maladie de Crohn, la gammapathie monoclonale, la granulo-matose de Wegener, le lupus, la maladie de Horton, la maladie de Reiter, la rectocolite hémorragique, le rhumatisme psoriasique, la sarcoïdose, la spondylarthrite ankylosante, les dermatites bulleuses auto-immunes, la colite collagène,, dermatite herpétiforme, fièvre méditerranéenne familiale, glomérulonéphrite à dépôts d'IgA, la glomérulonéphrite extra-membraneuse, l'hépatite auto-immune, le syndrome myasthénique de Lambert-Eaton, l'ophtalmie sympathique, la pemphigoïde bulleuse, le pemphigus, le purpura thrombopénique idiopathique, le syndrome de Fiessinger-Leroy-Reiter, la thyroïdite auto-immune, le syndrome uvéo-méningo-encéphalique et la dermatite herpétiforme.

Dans une autre illustration de la divulgation non revendiquée, les pathologies impliquant des cellules dendritiques plasmacytoïdes sont des maladies auto-immunes, de préférence des maladies auto-immunes caractérisées par une sécrétion accrue d'IFN de type I.

Dans un mode de réalisation avantageux, la divulgation non revendiquée concerne un anticorps monoclonal ou polyclonal dirigé contre la protéine BDCA-2 pour son utilisation selon la définition précédente, où les tumeurs hématopoïétiques sont:
- Les syndromes lymphoprolifératifs et myélome choisis parmi : maladie de Waldenstrôm, leucémie lymphoïde chronique, myélome multiple, amylose primitive,
- Les lymphomes choisis parmi : lymphome folliculaire, lymphome diffus, Lymphome de la zone marginale, maladie de Hodgkin,
- L'aplasie et les leucémies et myélodysplasies choisis parmi : leucémie aiguë lymphomblastique, leucémie aiguë myéloïde, myélodysplasie, aplasie médullaire,
- Les syndromes myéloprolifératifs choisis parmi: myélofibrose, polyglobulie primitive, thrombocythé-mie essentielle, leucémie myéloïde chronique.

Dans un mode de réalisation encore plus avantageux, l'invention concerne un anticorps monoclonal ou polyclonal dirigé contre la protéine BDCA-2 pour son utilisation selon la définition précédente, où ledit anticorps est choisi parmi les anticorps suivants : un anticorps murin, un anticorps chimérique, un anticorps humanisé et un anticorps humain.

Il est bien connu de l'homme de métier que les anticorps murins sont des anticorps produits par des cellules de souris, et les anticorps humains sont produits par des cellules humaines.
Toutefois, on généralisera dans l'invention les définitions précédentes d'anticorps humain et murin à tout anticorps comprenant des séquences d'acides aminés de leurs chaines lourdes et de leurs chaines légères d'homme ou des souris. Aussi, un anticorps murin est un anticorps dont les séquences des chaines lourdes et des chaines légères qui le constituent sont des séquences dont on retrouve la correspondance en acide nucléique dans le génome des cellules B murines. Cet anticorps est donc constitué de séquences d'acides aminés murines, quelle que soit l'origine de la cellule qui permet sa production. Par exemple, les séquences d'anticorps de souris exprimées dans des cellules de macaque donneront des anticorps murins.

La définition ci-dessus s'applique mutatis mutandis aux anticorps humains.

Par anticorps chimérique, on entend dans l'invention un anticorps isolé, dans lequel la séquence de chaque chaîne légère et/ou de chaque chaîne lourde qui le constitue comprend ou consiste en une séquence hybride issue d'au moins deux animaux distincts. Notamment les anticorps chimériques de l'invention sont des hybrides homme/macaque ou homme/souris, ce qui signifie qu'une région de la séquence des chaînes légères et des chaînes lourdes est issue de la séquence d'une immunoglobuline de macaque ou de souris, et que le reste de la séquence desdites chaînes lourdes et des dites chaînes légères est issue de la séquence d'une, ou éventuellement plusieurs, immunoglobuline humaine.

Par anticorps humanisé, on entend dans l'invention un anticorps issus d'un animal autre que l'homme dans lequel les séquences des chaines lourdes et des chaines légères autres que les CDR ont été remplacées par des séquences correspondantes d'un ou plusieurs anticorps d'origine humaines. L'anticorps est donc majoritairement constitué de séquences humaines, mais sa spécificité pour l'antigène conférée par les CDR est issue d'une autre espèce.

L'invention est aussi illustrée par un anticorps monoclonal ou polyclonal dirigé contre la protéine BDCA-2 pour son utilisation susmentionnée, les tumeurs sont des tumeurs solides choisies parmi le cancer du poumon, rein, foie, pancréas, le mélanome et l'ovaire.

Dans encore un autre mode de réalisation avantageux, l'invention concerne un anticorps monoclonal ou polyclonal dirigé contre la protéine BDCA-2 pour son utilisation susmentionnée, où ledit anticorps est un anticorps chimérique et de préférence un anticorps chimérique choisi parmi un anticorps chimérique murin/humain ou un anticorps chimérique humain/macaque

Dans encore un autre mode de réalisation avantageux, l'invention concerne un anticorps monoclonal ou polyclonal dirigé contre la protéine BDCA-2 dirigé pour son utilisation susmentionnée, où ledit anticorps est un anticorps humanisé.

Dans encore un autre mode de réalisation avantageux, l'invention concerne un anticorps monoclonal ou polyclonal dirigé contre la protéine BDCA-2 pour son utilisation susmentionnée, où ledit anticorps est un anticorps humain

Dans encore un autre mode de réalisation avantageux, l'invention concerne un anticorps monoclonal ou polyclonal dirigé contre la protéine dirigé contre la protéine BDCA-2 pour son utilisation susmentionnée, où ledit anticorps présente un taux de fucosylation inférieur à 60% des formes glycosylées.

Les anticorps selon l'invention présentent une faible quantité de fucose sur les chaines glycanniques portées par lesdits anticorps. Cette quantité de fucose, ou taux de fucose, est défini comme la proportion moyenne de fucose portée par tous les anticorps, par rapport à la quantité maximale de fucose que peuvent porter les chaines glycanniques.

Un autre aspect de la divulgation non revendiqué concerne des fragments d'un anticorps monoclonal ou polyclonal dirigé contre la protéine BDCA-2 pour son utilisation telle que définie précédemment.

Le terme « fragment d'anticorps » définit une portion d'anticorps, de préférence un site de liaison à l'antigène ou une région variable de l'anticorps. Des exemples de fragments incluent Fv, Fab, F(ab')2, Fab', dsFv, scFv, sc(Fv)2, diabody, triabody ou tetrabody, et des anticorps multispécifiques composés de différents fragments. Dans encore un autre mode de réalisation avantageux, la divulgation non revendiquée concerne ainsi des fragments d'un anticorps monoclonal ou polyclonal dirigé contre la protéine BDCA-2 pour son utilisation susmentionnée, où ledit fragment est choisi parmi les Fab, F(ab)'2, Fd, scFV, dimère de ScFv, diabody, triabody ou tetrabody.
L'ensemble de ces fragments d'anticorps sont bien connus de l'homme de métier.
Pour exemples, quelques définitions sont données ci-après.
Le terme « Fab » désigne un fragment d'anticorps de masse moléculaire d'environ 50.000 dalton et possédant une activité de liaison à l'antigène. Il comprend environ la moitié du côté N-terminal de la chaîne lourde et la chaîne légère entière liés par un pont disulfure. Le Fab peut être obtenu notamment par le traitement des IgG par une protéase, la papaïne.

Le terme « F(ab')2 » désigne un fragment d'environ 100.000 dalton et une activité de liaison à l'antigène, l'association par un pont disulfure de deux Fab décrits ci-dessus. Il peut être obtenu par le traitement des IgG par une protéase, la pepsine.

Un « scFv » (single chain Fv) est un polypeptide VH:VL synthétisé en utilisant les gènes codant pour les domaines VL et VH et une séquence codant pour un peptide destiné à lier ces domaines. Un scFv selon l'invention inclut les CDR maintenus dans une conformation appropriée, par exemple en utilisant des techniques de recombinaison génétique.
Les dimères de ScFv correspondent à deux molécules de scFv reliées entre elles par une liaison peptidique. Les ScFv peuvent également servir de modules de base pour le développement de structures multimérique (dimerique : « diabody », trimérique : « triabody », tétramerique : « tetrabody »). Ainsi, le *diabody* est un dimère de scFv. La séquence de fixation est plus courte, et donc ne permettra pas l'auto-association entre les chaînes lourdes et légères dans un scFv solitaire. Ce dimère de fragment a la propriété supplémentaire de maintenir la double valence que l'anticorps parent possède.
Aussi, dans la divulgation non revendiquée, on entend par « triabody » l'association trivalente de scFv, ledit triabody pouvant fixer 3 antigènes identiques ou différents.
Dans la divulgation non revendiquée, un « tétrabody » correspond à l'association tétravalente de scFv, ledit tetrabody et pouvant fixer 4 antigènes identiques ou différents.

Un autre objet de la divulgation non revendiqué est un anticorps ou fragment d'anticorps, où ledit anticorps ou fragment d'anticorps est couplé à une molécule bioactive choisi parmi les radio-isotopes, les métaux non-radioactifs, les toxines, les acides nucléiques, les agents cytotoxiques ou encore les enzymes.
La divulgation non revendiquée ainsi un anticorps monoclonal ou fragment d'anticorps monoclonal tels que définis précédemment, où ledit anticorps ou fragment d'anticorps est couplé à une molécule bioactive choisi parmi les radio isotopes, les métaux non-radioactifs, les toxines (choisies parmi la ricine, l'abrine, la toxine diphtérique), les acides nucléiques choisis parmi les ARNs anti-sens, les agents cytotoxiques choisis parmi la mitomycine C, le méthotrexate, l'adriamycine, les enzymes telles que les RNases, la biotine, l'avidine ou la streptavidine.

De tels anticorps couplés à une molécule bioactive sont capable d'adresser spécifiquement un radio isotope, une enzyme, un métal lourd, ou encore une toxine, greffé audit anticorps à une cellule cible déterminée, en l'occurrence les cellules DC plasmacytoïdes.
On peut citer comme isotopes avantageux les radio-isotopes suivants At211, I131, I125, Y90, Re186, Re188, Sm153, Bi212, P32, cette liste n'étant pas limitative. Les toxines que l'on peut greffer aux anticorps selon l'invention sont choisies parmi la ricine, la toxine tétanique, l'abrine, la toxine diphtérique.
Les agents cytotoxiques choisis parmi les antifolates (méthotrexate, pémétrexed, raltitrexed) les anti-purines (cladribine, fludarabine, azathioprine, azathioprine, mercaptopurine, 5-fluorouracile capécitabine, cytarabine, gemcitabine), les inhibiteurs de topoisomérases I et II, les agents alkylants (chlorméthine, cyclophosphamide, ifosfamide, carmustine, fotémustine) et apparentés (mi-tomycine C, cisplatine, carboplatine, oxaliplatine), les agents intercalants, les anthracyclines (daunorubicine, doxorubicine et chlorydrate, epirubicine, idarubicine, bléomycine), les taxanes, les inhibiteurs spécifiques de tyrosine-kinase (imatinib, Erlotinib), peuvent également être utilisés. L'ensemble de ces molécules ou composés que l'on peut greffer sur les anticorps selon l'invention sont bien connus de l'homme de métier, et il lui est aisé de déterminer la molécule à utiliser.
Dans un aspect avantageux, la divulgation non revendiquée concerne ainsi un anticorps monoclonal ou polyclonal tel que précédemment défini ou un fragment d'anticorps monoclonal tel que précédemment défini, où ledit anticorps ou ledit fragment d'anticorps est couplé à une molécule bioactive choisie parmi:
- les radio-isotopes,
- les métaux non-radioactifs,
- les toxines choisies parmi la ricine, l'abrine, la toxine diphtérique,
- les acides nucléiques choisis parmi les ARNs anti-sens,
- les agents cytotoxiques choisis parmi:
   - les antifolates choisis parmi méthotrexate, pémétrexed, raltitrexed,
   - les anti-purines choisies parmi : cladribine, flu-darabine, azathioprine, azathioprine, mercapto-purine, 5-fluorouracile capécitabine, cytarabine, gemcitabine,
   - les inhibiteurs de topoisomérases I et II,
   - les agents alkylants choisis parmi : chlorméthine, cyclophosphamide, ifosfamide, carmustine, fotémustine et les agents alkylants apparentés choisis parmi : mitomycine C, cisplatine, carbo-platine, oxaliplatine,
   - les agents intercalants,
   - les anthracyclines choisies parmi : daunorubicine, doxorubicine et chlorydrate, epirubicine, idarubicine, bléomycine,
   - les taxanes,
   - les inhibiteurs spécifiques de tyrosinekinase choisis parmi : imatinib, Erlotinib,

   - les enzymes telles que les RNases,
   - la biotine, l'avidine ou la streptavidine.

L'invention concerne également un produit de combinaison comprenant un anticorps susmentionné, en association avec un agent anticancéreux, pour une utilisation simultanée, séparée ou étalée dans le temps, en thérapie tumorale.

L'invention concerne ainsi un produit de combinaison comprenant un anticorps tel que précédemment défini, et un agent anticancéreux pour une utilisation simultanée, séparée ou étalée dans le temps en thérapie tumorale, dans le traitement des tumeurs hématopoïétiques de phénotype CD4+, CD56+.

Les anticancéreux pouvant être utilisés dans l'invention sont notamment choisis parmi
- les anti-métaboliques tels que les antifoliques (méthotrexate, raltitrexed, et pemetrexed), les antipuriques (mercaptopurine, thioguanine, pentostatine, cladribine et fludarabine), les antipyrimidiques (5 fluoro-uracile, tégafur uracile, capécitabine et cytarabine), et autres anti-métaboliques (hydroxycarbamide ou hydroxyurée et gemcitabine),
- les alkylants tels que les moutardes à l'azote (chlorambucil, melphelan, chlorméthine ou métachloroéthamine, estramustine, ifosfamide et cyclophosphamide), les nitroso-urées (fotémustine, lomustine, carmustine et streptozocine), les organoplatines (carboplatine, cisplatine et oxaliplatine), les ethylène imines (thiotépa et altrétamine), les triazènes (procarbazine, témozolomide et dacarbazine) et d'autres alkylants (busulfan, mitomycine C et pipobroman),
   -- les agents intercalant tels que les dérivés de la camptothécine (irinotécan et topotécan) les antrhra-cyclines (epirubicine, daunorubicine, doxorubicine, pirarubicine et idarubicine) et d'autres agents intercalant (mitoxantrone, amsacrine, elliptinium, actino-mycine d ou dactinomycine, etoposide et bléomycine), et
   -- les molécules ayant une action sur le fuseau mitotique telles que les vinca-alcaloïdes ou poisons du fuseau (vinorelbine, vindésine, vincristine et vinblastine), les taxoïdes ou stabilisants du fuseau (paclitaxel et docétaxel), les inhibiteurs de tyrosine kinase (dasatinib, erlotinib, imatinib, sorafénib et sunitinib).

Les molécules susmentionnées sont données à titre indicatif mais ne sauraient limiter la portée de l'invention. Les composés susmentionnés correspondent à la dénomination commune internationale (DCI) et l'homme de métier peut très facilement retrouver les marques commerciales correspondantes chez les différents fournisseurs.

L'invention concerne un produit de combinaison susmentionné pour une utilisation dans le traitement des tumeurs hématopoïétiques.
L'invention concerne un produit de combinaison susmentionné, pour son utilisation dans le traitement des tumeurs de phénotype CD4+, CD56+.

### LEGENDE DES FIGURES

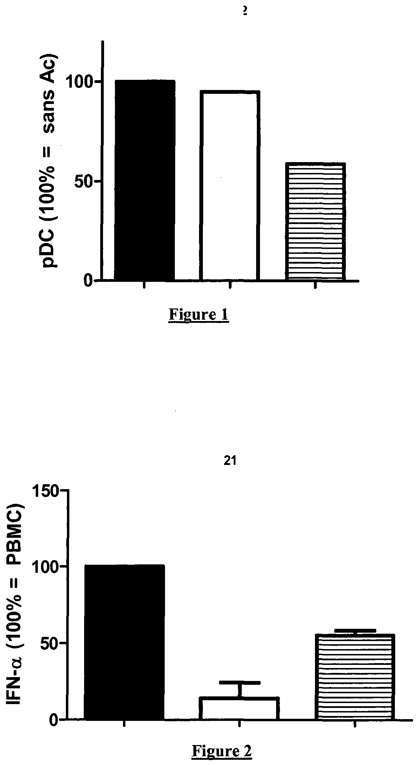

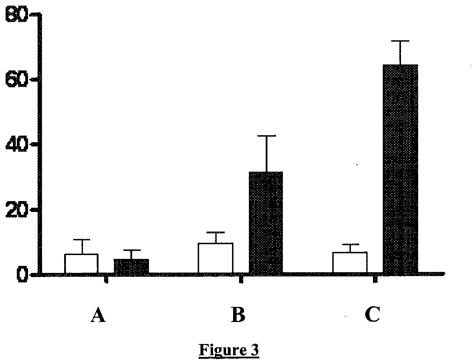

**La** **figure 1** représente un histogramme montrant la déplétion des cellules DC plasmacytoïdes BDCA-2+ dans des PBMC humaines de donneur sain en présence de l'anticorps polyclonal de lapin anti-BDCA-2+. Le % de cellules positives est obtenu par un double marquage ILT7/BDCA-2.
   La colonne noire représente le pourcentage de cellule cellules DC plasmacytoïdes sans traitement avec l'anticorps (contrôle négatif 1), la colonne blanche représente le pourcentage de cellule cellules DC plasmacytoïdes traitées avec un anticorps contrôle (qui n'est pas dirigé contre BDCA-2 ; contrôle négatif 2) et la colonne hachurée représente le pourcentage de cellule cellules DC plasmacytoïdes traitées l'anticorps anti BDCA-2.
**La** **figure 2** représente un histogramme montrant le dosage de l'IFN-α après déplétion des cellules DC plasmacytoïdes avec un anticorps polyclonal de lapin anti-BDCA-2+ et activation par du CpG.
   La colonne noire représente le pourcentage de sécrétion d'IFN-α par les PBMC totaux, la colonne blanche représente le pourcentage de sécrétion d'IFN-α par les PBMC totaux dépélété en cellules DC plasmacytoïdes et la colonne hachurée représente le pourcentage de sécrétion d'IFN-α par les cellules DC plasmacytoïdes seules.
**La** **figure 3** représente un histogramme montrant l'activité ADCC de l'anticorps polyclonal anti-BDCA-2 (barres noires) sur les cellules Jurkat-BDCA-2. Les résultats sont exprimés en pourcentage de lyse de la cellule Jurkat-BDCA-2 en fonction de la quantité d'anticorps ajoutée, exprimé en facteur de dilution de la solution initiale d'anticorps (A : 0, B : 1/250 et C : 1/25). Moyenne +/- écart type. En contrôle, le pourcentage de lyse est mesuré à l'aide d'un anticorps contrôle (barres blanches). L'axe des Y représente le pourcentage de lyse.

### EXEMPLES

### Exemple 1 : préparation de lignées cellulaires exprimant la protéine BDCA-2.

L'ADN complémentaire de BDCA-2 est amplifié par PCR et cloné clonée dans le vecteur d'expression pcDNA3.1(-) aux sites de restriction HindIII/BamHI.

Le vecteur pcDNA contenant BDCA-2 est transfecté par nucléofection ou Fugene HD la lignée cellulaire U937 ou THP-1.

48h heures après transfection, l'expression de BDCA-2 par les cellules transfectées est suivie par cytométrie en flux, en utilisant un anticorps anti-BDCA-2 et un anticorps couplé à un fluorophore.

Les cellules BDCA-2+ seront triées par sélection positive à l'aide de billes magnétiques couplées à l'anticorps anti-BDCA-2 (kit myltenyi).

Le tri est répété jusqu'à obtention de 100% de cellules positives exprimant BDCA-2.

### Exemple 2 : Mesure de l'effet de l'anticorps anti-BDCA-2 selon l'invention.

L'effet de l'anticorps anti-BDCA-2 selon l'invention est mesuré chez la souris ou chez l'homme selon sa nature.

Un moyen de mesurer l'activité de l'anticorps est de mesurer la lyse cellulaire dépendante des anticorps (ADCC) en présence de cellules tueuses (Natural Killer (NK) ou lignées T transfectées avec des récepteurs Fc).

### Mesure de l'ADCC pour un anticorps murin ou présentant un fragment Fc d'origine murine

Les cellules tueuses (cellules effectrices de souris ou des lignées T transfectées avec des FcR murins) sont incubées avec des cellules cibles transfectées BDCA-2 (exemple 1) ou des cellules dendritiques plasmacytoïdes de patients leucémiques (CD4+/CD56+) ou atteints de maladie inflammatoire et/ou auto-immune, dans un ratio E/T de 15/1, en présence de différentes concentrations d'anticorps anti-BDCA-2.

Après 4 ou 16 heures d'incubation, l'activité cytotoxique induite par les anticorps anti-BDCA-2 est mesurée par colorimétrie en dosant dans les surnageants la libération de la lactate déshydrogénase (LDH) (Roche Diagnostics - Cytotoxicity Detection Kit LDH réf 11644793001).

En cas de faible taux de LDH intra-cellulaire, les cellules sont marquées avec un agent fluorescent et la lyse est estimée en mesurant la quantité de fluorescence relarguée dans le surnageant.

Les résultats de lyse spécifique sont exprimés en pourcentage de lyse en fonction de la concentration d'anticorps. Les valeurs d'EC₅₀ (quantité d'anticorps induisant 50% de la lyse maximale) ainsi que les valeurs d'Eₘₐₓ (pourcentage de lyse maximal) sont calculées à l'aide du logiciel PRISM.

### Mesure de l'ADCC pour un anticorps possédant un fragment Fc d'origine humaine

Les cellules tueuses (PBMC, cellules NK, ou cellules T transfectées avec du CD16 humain) sont préalablement purifiées par la technique de déplétion négative développée par la société Miltenyi (Miltenyi Biotec - NK cell isolation kit human réf 130-092-657), à partir de sang périphérique de donneurs sains. La technique ADCC consiste à incuber les cellules NK avec des cellules cibles transfectées BDCA-2 ou des cellules DC plasmacytoïdes de patients leucémiques (CD4+/CD56+) ou atteints de maladie inflammatoire et/ou auto-immune, à un ratio E/T de 15/1, en présence de différentes concentrations d'anticorps anti-BDCA-2.

Après 4 ou 16 heures d'incubation, l'activité cytotoxique induite par les anticorps anti-BDCA-2 est mesurée par colorimétrie en dosant dans les surnageants, une enzyme intracellulaire nommée lactate déshydrogénase (LDH) libérée par les cellules cibles lysées (Roche Diagnostics - Cytotoxicity Detection Kit LDH réf 11644793001).

En cas de faible taux de LDH intra-cellulaire, les cellules sont marquées avec un agent fluorescent et la lyse est estimée en mesurant la quantité de fluorescence relarguée dans le surnageant.

Les résultats de lyse spécifique sont exprimés en pourcentage de lyse en fonction de la concentration d'anticorps. Les valeurs d'EC₅₀ (quantité d'anticorps induisant 50% de la lyse maximale) ainsi que les valeurs d'Eₘₐₓ (pourcentage de lyse maximal) sont calculées à l'aide du logiciel PRISM.

### Exemple 3 : préparation de lignées cellulaires exprimant la protéine BDCA-2.

L'ADN complémentaire de BDCA-2 est amplifié par PCR et cloné clonée dans le vecteur d'expression pcDNA3.1(-) aux sites de restriction HindIII/BamHI. Le vecteur pcDNA contenant BDCA-2 est transfecté par nucléofection ou Fugene HD la lignée cellulaire Jurkat. 48h heures après transfection, l'expression de BDCA-2 par les cellules transfectées est suivie par cytométrie en flux, en utilisant un anticorps anti-BDCA-2 couplé à un fluorophore. Les cellules BDCA-2+ seront isolées à l'aide de tris successifs réalisés par cytométrie en flux avec un trieur (Altra, Becton Dickson) en utilisant un anticorps anti BDCA-2 (Myltenyi). Le tri est répété jusqu'à obtention de 100% de cellules positives exprimant BDCA-2.

### Exemple 4 : Cytotoxicité.

### Déplétion de cellules DC plasmacytoïdes dans des PBMC

Les PBMC sont isolés à partir du sang périphérique sur un gradient de Ficoll. La technique consiste à incuber les PBMC en présence d'un anticorps polyclonal de lapin anti BDCA-2 ou d'anticorps polyclonal de lapin irrelevant. Après 16 heures d'incubation, la dépletion des cellules DC plasmacytoïdes induite par les anticorps anti BDCA-2 est mesurée par cytométrie en flux sur la base du double marquage ILT7/CD123.

Les résultats sont montrés dans la figure 1.

Ces résultats montrent une diminution du nombre de cellules DC plasmacytoïdes d'environ 40% en présence d'anticorps anti-BDCA-2 en comparaison à l'anticorps contrôle. Ce résultat permet de conclure qu'un anticorps anti-BDCA-2, en présence de cellules effectrices, peut induire une déplétion des cellules DC plasmacytoïdes.

### Diminution du taux d'IFNα associé à la déplétion des cellules DC plasmacyloïdes

Les PBMC sont isolés à partir du sang périphérique sur un gradient de Ficoll. La technique consiste à dépléter les cellules DC plasmacytoïdes des PBMC à l'aide du kit Myltenyi anti-cellules DC plasmacytoïdes. Après activation par CpG pendant 16h à 37°C, le taux d'IFN-α est mesuré par cytométrie en flux et cela en comparaison avec les PBMC témoin contenant des cellules DC plasmacytoïdes.

Les résultats sont montrés dans la figure 2.

Les résultats indiquent une diminution de la sécrétion d'IFN-α après déplétion des cellules DC plasmacytoïdes d'environ 85%, ceci permettant de conclure que la déplétion des cellules DC plasmacytoïdes par un anti-BDCA-2 peut conduire à une diminution de la sécrétion d'IFN-α. Dans cette expérience, les cellules DC plasmacytoïdes seules sont utilisées en tant que cellules positives pour la sécrétion d'IFN-α.

### ADCC

Les cellules tueuses (cellules NK) sont préalablement purifiées par la technique de déplétion négative développée par la société Miltenyi (Miltenyi Biotec - NK cell isolation kit human réf 130-092-657), à partir de sang périphérique de donneurs sains. La technique ADCC consiste à incuber les cellules NK avec des cellules cibles de la lignée Jurkat transfectées avec le récepteur BDCA-2, en présence de différentes concentrations d'un anticorps polyclonal de lapin anti-BDCA-2 ou d'anticorps polyclonal de lapin irrelevant. Après 16 heures d'incubation, l'activité cytotoxique induite par les anticorps anti-BDCA-2 est mesurée par colorimétrie en dosant dans les surnageants, une enzyme intracellulaire nommée lactate déshydrogénase (LDH) libérée par les cellules cibles lysées (Roche Diagnostics - Cytotoxicity Detection Kit LDH).

Les résultats sont présentés dans la figure 3 et montre une activité cytotoxique de l'anticorps polyclonal de lapin anti-BDCA-2 sur les cellules Jurkat-BDCA-2, de manière dose dépendante pouvant atteindre plus de 60% de lyse de la cellule cible.

Ces résultats indiquent donc qu'en ciblant l'antigène BDCA-2 exprimé à la surface d'une cellule, il est possible de lyser cette cellule en utilisant des effecteurs tels que les cellules NK. L'anticorps anti-BDCA-2 peut supporter d'autres activités cytotoxiques telles que la phagocytose dépendante d'autres cellules effectrices comme les macrophages ou les neutrophiles.

## Revendications

1. Anticorps monoclonal ou polyclonal dirigé contre la protéine BDCA-2 pour son utilisation dans le cadre de la prévention ou du traitement des pathologies impliquant une activation des cellules dendritiques plasmacytoïdes, où lesdites pathologies sont des tumeurs hématopoïétiques de phénotype CD4+, CD56+, ledit anticorps impliquant une destruction des cellules DC plasmacytoïdes par apoptose ou par le mécanisme de l'ADCC.

2. Anticorps monoclonal ou polyclonal dirigé contre la protéine BDCA-2 pour son utilisation selon la revendication 1, où ledit anticorps est choisi parmi les anticorps suivants : un anticorps murin, un anticorps chimérique, un anticorps humanisé et un anticorps humain.

3. Anticorps monoclonal ou polyclonal dirigé contre la protéine BDCA-2 pour son utilisation selon la revendication 2, où ledit anticorps est un anticorps chimérique et de préférence un anticorps chimérique choisi parmi un anticorps chimérique murin/humain ou un anticorps chimérique humain/macaque

4. Anticorps monoclonal ou polyclonal dirigé contre la protéine BDCA-2 pour son utilisation selon la revendication 2, où ledit anticorps est un anticorps humanisé.

5. Anticorps monoclonal dirigé contre la protéine BDCA-2 pour son utilisation selon la revendication 2, où ledit anticorps est un anticorps humain

6. Anticorps monoclonal ou polyclonal dirigé contre la protéine BDCA-2 pour son utilisation selon l'une quelconque des revendications précédentes, où ledit anticorps présente un taux de fucosylation inférieur à 60% des formes glycosylées.

7. Produit de combinaison comprenant un anticorps tel qu'il est défini dans l'une quelconque des revendications 1 à 6, et un agent anticancéreux pour une utilisation simultanée, séparée ou étalée dans le temps en thérapie tumorale, dans le traitement des tumeurs hématopoïétiques de phénotype CD4+, CD56+.

## Patentansprüche

1. Monoklonaler oder polyklonaler Antikörper, der gegen das BDCA-2 Protein gerichtet ist, zur Verwendung im Zusammenhang mit der Prävention oder Behandlung von Krankheiten, die eine Aktivierung von plasmazytoiden dendritischen Zellen mit sich bringen, wobei die Krankheiten hämatopoetische Tumore des Phänotyps CD4+, CD56+ sind, wobei der Antikörper eine Zerstörung der plasmazytoiden DC-Zellen durch Apoptose oder durch den Mechanismus der ADCC einschliesst.

2. Monoklonaler oder polyklonaler Antikörper, der gegen das BDCA-2 Protein gerichtet ist, zur Verwendung nach Anspruch 1, wobei der Antikörper aus den folgenden Antikörpern ausgewählt wird: einem murinen Antikörper, einem chimären Antikörper, einem humanisierten Antikörper und einem humanen Antikörper.

3. Monoklonaler oder polyklonaler Antikörper, der gegen das BDCA-2 Protein gerichtet ist, zur Verwendung nach Anspruch - 2, wobei der Antikörper ein chimärer Antikörper ist und vorzugsweise ein chimärer Antikörper, ausgewählt aus einem chimären murinen/humanen Antikörper oder einem chimären humanen/Makaken-Antikörper.

4. Monoklonaler oder polyklonaler Antikörper, der gegen das BDCA-2 Protein gerichtet ist, zur Verwendung nach Anspruch 2, wobei der Antikörper ein humanisierter Antikärper ist.

5. Monoklonaler Antikörper, der gegen das BDCA-2 Protein gerichtet ist, zur Verwendung nach Anspruch 2, wobei der Antikörper ein humaner Antikörper ist.

6. Monoklonaler oder polyklonaler Antikörper, der gegen das BDCA-2 Protein gerichtet ist, zur Verwendung nach einem der vorherigen Ansprüche, wobei der Antikörper eine Fukosylierungsrate von weniger als 60% glycosylierter Formen aufweist.

7. Kombinationsprodukt, das einen Antikörper wie in einem der Ansprüche 1 bis 6 definiert umfasst, sowie ein Antikrebsmittel, zur gleichzeitigen, getrennten oder langanhaltenden Verwendung bei der Tumortherapie, bei der Behandlung von hämatopoetischen Tumoren des Phänotyps CD4+, CD56+.

## Claims

1. Monoclonal or polyclonal antibody directed against the BDCA-2 protein for its use in the prevention or treatment of pathologies involving activation of the plasmacytoid dendritic cells, where said pathologies are haematopoietic tumours of phenotype CD4+, CD56+, said antibody involving the destruction of the plasmacytoid DC cells by apoptosis or by the ADCC mechanism.

2. Monoclonal or polyclonal antibody directed against the BDCA-2 protein for its use according to claim 1, where said antibody is chosen from the following antibodies: a murine antibody, a chimeric antibody, a humanized antibody and a human antibody.

3. Monoclonal or polyclonal antibody directed against the BDCA-2 protein for its use according to claim 2, where said antibody is a chimeric antibody and preferably a chimeric antibody chosen from a murine/human chimeric antibody or a human/macaque chimeric antibody.

4. Monoclonal or polyclonal antibody directed against the BDCA-2 protein for its use according to claim 2, where said antibody is a humanized antibody.

5. Monoclonal antibody directed against the BDCA-2 protein for its use according to claim 3, where said antibody is a human antibody.

6. Monoclonal or polyclonal antibody directed against the BDCA-2 protein for its use according to any one of the preceding claims, where said antibody has a fucosylation level of less than 60% of the glycosylated forms.

7. Combination product comprising an antibody as defined in any one of claims 1 to 6, and an antineoplastic agent for a use which is simultaneous, separate or spread over time, in tumour therapy, in the treatment of haematopoietic tumours of phenotype CD4+, CD56+.
